# NEUE EUROPÄISCHE PATENTSCHRIFT

(11) **EP 0 632 965 B2**
(45) Veröffentlichungstag und Bekanntmachung des Hinweises auf die Entscheidung über den Einspruch: **20.03.2002**
(45) Hinweis auf die Patenterteilung: 17.06.1998
(21) Anmeldenummer: 94110243.6
(22) Anmeldetag: 01.07.1994
(51) Int. Cl.: A23L 3/08, A61L 2/24, A61L 2/06

(54) **Verfahren zum mehrstufigen Behandeln von stückigen Produkten mittels Prozessmedien und Vorrichtung zur Durchführung des Verfahrens**
Process and apparatus for the stepwise treatment of lump products with process fluids
Procédé et dispositif de traitement des produits en morceaux à plusieurs stades avec des fluides de traitement

(30) Priorität: 06.07.1993 DE 4322467
(43) Veröffentlichungstag der Anmeldung: 11.01.1995
(73) Patentinhaber: Fresenius AG, 61350 Bad Homburg (DE)
(72) Erfinder: Heilmann, Klaus, D-66606 St. Wendel (DE); Lauer, Martin, D-66606 St.Wendel (DE)
(74) Vertreter: Luderschmidt, Schüler & Partner GbR

(56) Entgegenhaltungen:
- DE-A- 3 002 610
- FR-A- 2 191 907
- GB-A- 2 055 289

## Beschreibung

Die Erfindung bezieht sich auf ein Verfahren zum Behandeln von stückigen Produkten mittels Prozeßmedien im Rahmen einer Abfolge von Verfahrensschritten (Behandlungsstufen) in einer Behandlungseinheit. Sie bezieht sich ferner auf eine entsprechende Vorrichtung zum Behandeln von stückigen Produkten mittels einer Versorgungseinheit entnehmbarer Prozeßmedien im Rahmen einer Abfolge von Verfahrensschritten (Behandlungsstufen) in einer Behandlungseinheit.

Ein derartiges Verfahren einschließlich der zugehörigen Vorrichtung ist durch die EP-A1 0 333 389 bekannt.

Dieser Stand der Technik beschreibt einen sogenannten Großraumautoklav, der zur Durchführung der meisten Behandlungsverfahren, bei denen unterdruck, bzw. schädliche Medien vorherrschen, eingesetzt wird.

Dieser Großraumautoklav weist eine verhältnismäßig große verschließbare Kammer auf, in welche die zu behandelnden Produkte chargenweise eingebracht werden.

In der Kammer werden dann zeitlich nacheinander die verschiedenen Behandlungsprozesse mit ihren charakteristischen Parametern wie Medienart, Durchfluß, Temperatur und Druck an allen in der Kammer befindlichen Produkten gleichzeitig durchgeführt.

Solche Einkammerautoklaven, mit denen nach dem Batchverfahren gearbeitet wird, sind in der Anschaffung und im Betrieb umso günstiger, je größer sie gebaut werden. In der Anschaffung deshalb, weil die Vergrößerung der Kammer und der Aggregate billiger ist als die Realisierung mehrerer kleiner Autoklavanlagen; im Betrieb deshalb, weil bei thermischen Umpolungsvorgängen die mitumzupolende Anlagenmasse bei mehreren kleinen Autoklaven größer ist als bei einem großen Autoklav. Außerdem ist bei mehreren kleinen Autoklaven beim heute üblichen Automatisierungsgrad der Organisations- und Personalaufwand größer als beim Großraumautoklaven.

Der Großraumautoklav weist einige gravierende Nachteile auf.
1. Die Gleichmäßigkeit der Behandlung zwischen den Produkten, die in einer Kammer behandelt wurden, d.h. die Produktqualität, wird im allgemeinen um so geringer, je mehr Produkte in die Kammer passen, d.h. in einem Zyklus autoklaviert werden. Durch die Vielzahl der Produkte kann nämlich die Gleichmäßigkeit der Temperatur- und Druckverhältnisse innerhalb der Kammer nicht mehr garantiert werden. Die anschaffungsmäßige Überlegenheit der Großraumautoklaven beruht aber gerade darauf, den für die Produkte zur Verfügung stehenden Raum und die Medienführungsquerschnitte in geringerem Maße anwachsen zu lassen als dies der Fall wäre, wenn man zur Erzielung der gleichen Produktivität mehrere kleine Autoklaven verwenden würde. Die geringere Gleichmäßigkeit macht zur Vermeidung einer zu großen Menge von Ausschußware eine Überdimensionierung des Behandlungsverfahrens notwendig; dies wiederum verschlechtert die Produktqualität, die Produktivität und den Energieverbrauch und erhöht den Aufwand für die laufenden Qualitätskontrollen.
2. Die hohe Anzahl der in der Kammer des Großraumautoklaven gleichzeitig zu behandlenden Produkte verlangt, daß vor und nach der Autoklavanlage entsprechend groß dimensionierte Pufferläger für die zu behandelnden und für die behandelten Produkte eingerichtet und betrieben werden müssen. Der Aufwand für einen vollautomatisierten Betrieb dieser Puffer ist beträchtlich.
3. Durch den Chargen- oder Batch-Betrieb ist während der Bestückung sowie während des Abkühlens und Ausräumens der behandelten Produkte kein Autoklavieren möglich. Bei einer Großzahl von Produkten wird daher die Effektivität einer solchen Großraumautoklav-Anlage geringer.

Um diese Probleme zu umgehen, ist man gerade bei Lebensmitteln oder Infusionsflaschen dazu übergegangen, kontinuierlich im Durchlaufverfahren zu behandeln, insbesondere zu sterilisieren. Diese Sterilisationsverfahren sind in einer Reihe von Patentschriften und Literaturstellen beschrieben worden, wobei stellvertretend auf die US-A 4,094,633, die EP A1 0 427 323 und das Buch von Wallhäuser, Praxis der Sterilisation (Seiten 242/243) Bezug genommen wird.

Allen Verfahren gemeinsam ist die Sterilisation der Produkte im Durchlauf durch einen Raum (Hitzekammer), der das sterilisierende Medium (z. B. Wasserdampf) enthält. Unter Druck ist eine solche Anlage nur zu betreiben, wenn die Hitzekammer mit Schleusen oder Ventilen ausgestattet ist, wie dieses in der US 4,094,633 beschrieben ist.

Die kontinuierlichen Anlagen arbeiten meistens nach einem 3-Phasen-System: Vorwärmen - Sterilisieren - Kühlen, wobei die einzelnen Vorgänge in der Regel in verschiedenen, miteinander verbundenen, ortsfesten Kammern ablaufen, die in Form eines Turmes angeordnet sein können, d.h. jedes Produkt durchläuft jede Behandlungsstufe zeitlich nacheinander in einer anderen Kammer (Behandlungseinheit).

Alle kontinuierlich arbeitenden Anlagen weisen den Nachteil auf, daß sie sehr auf das Produkt spezialisiert sind. Da sie der Rentabilität wegen automatisch bestückt werden, ist diese Zuführanlage ebenfalls speziell auf den Autoklaven ausgerichtet. Eine solche Anlage ist beispielsweise in der US 4,015,935 beschrieben.

Ein weiterer Nachteil besteht darin, daß in einer solchen Anlage stets das gleiche Sterilisationsmedium verwendet werden muß.

Aus der DE 35 07 337 C2 ist ein weiteres Mehrkammer-System bekannt geworden. Sie zeigt ein Verfahren sowie eine zugehörige Vorrichtung zum Beschichten von Substraten im Rahmen einer Abfolge von Verfahrensschritten. Für jeden Verfahrensschritt (Behandlungsstufe) ist ein Gruppe von Behandlungs-Behältern vorgesehen, die sowohl linear als auch zirkular angeordnet werden können. Mittels eines Transportsystems, das eine Transportkammer mit Schleuse aufweist, werden die Substrate nach der Behandlung mit dem Verfahrensschritt "n" aus dem Behälter der zugehörigen Gruppe in die Transportkammer ausgeschleust und von dort aus in einen Behälter der Gruppe, die dem Verfahrensschritt "n+1" zugeordnet ist, eingeschleust.

Bei diesem System laufen daher die verschiedenen Verfahrensschritte in verschiedenen Kammern, wobei die zu behandelnden Güter von Kammer zu Kammer transportiert werden, im Gegensatz zur Autoklav-Technik, bei der das Gut in der Kammer verbleibt und dort den einzelnen Verfahrensschritten nacheinander ausgesetzt wird.

Das bekannte System erfordert daher ein zeit- und materialaufwendiges Transport- und Schleusensystem. Es ist zudem speziell auf das Beschichten von Substraten ausgelegt.

Der Erfindung liegt die Aufgabe zugrunde, das eingangs bezeichnete Verfahren bzw. die entsprechende Vorrichtung so weiterzubilden, daß - unter Beibehaltung der Batch-Betriebsweise - die Produktqualität verbessert wird, Pufferläger weitgehend entfallen und trotz Beibehaltung der Batch-Betriebsweise eine quasi-kontinuierliche Behandlung möglich ist.

Die Lösung dieser Aufgabe gelingt für das Verfahren dadurch, daß als Behandlungseinheit eine Kette von mindestens drei getrennten Behandlungs-Einzeleinheiten gebildet wird, in denen die stückigen Produkte für die Dauer der Abfolge der Verfahrensschritte verbleiben, daß in der Kette, aufgeteilt auf die einzelnen Behandlungs-Einzeleinheiten, die einzelnen Verfahrensschritte der Abfolge gleichzeitig ablaufen, daß die Abfolge der Verfahrensschritte in den Behandlungs-Einzeleinheiten der Kette bzw. in Gruppen davon, in einem vorgegebenen Takt zeitlich versetzt beginnt, und daß der zeitliche Versatz (Takt) von einer gemeinsamen Steuer- bzw. Regeleinheit vorgegeben wird.

Hinsichtlich der Vorrichtung besteht die Lösung der Aufgabe darin, daß die Behandlungseinheit aus einer Mehrzahl von mindestens drei separaten Behandlungs-Einzeleinheiten besteht, die jeweils mindestens einen Zu- und Abfluß für die Prozeßmedien aufweisen, und in denen die stückigen Produkte für die Dauer der Abfolge der Verfahrensschritte verbleiben, daß die Zu- und Abflüsse der Behandlungs-Einzeleinheiten mit der Versorgungseinheit so verbunden sind, daß die Kette, aufgeteilt auf die einzelnen Behandlungs-Einzeleinheiten, die einzelnen Verfahrensschritte der Abfolge gleichzeitig ablaufen, daß eine gemeinsame Steuer- bzw. Regeleinheit vorgesehen ist, die derart aufgebaut und mit den Behandlungs-Einzeleinheiten verbunden ist, daß die Abfolge der Verfahrensschritte in den Behandlungs-Einzeleinheiten der Kette bzw. Gruppen davon, in einem vorgegebenen Takt zeitlich versetzt beginnt.

Die Behandlungs-Einzeleinheit kann dabei gebildet werden durch:
- eine Kammer mit einem Stückgut,
- eine Kammer mit kleiner Charge,
- ein zu behandelndes Einzelstück, welches (ohne umgebende Kammerwand) mit Konnektoranschlüssen befestigt ist.

Vorzugsweise wird die gemeinsame Steuereinheit mechanisch in Form eines Transportsystems für die Behandlungs-Einzeleinheiten gebildet, das die Behandlungs-Einzeleinheiten an verschiedene Orte transportiert, in denen jeweils mindestens eine Behandlungsstufe durchgeführt wird, und der Beginn der Behandlung durch den Ort der jeweiligen Kammer festgelegt bzw. ist den Behandlungseinheiten als gemeinsame Steuereinheit eine getaktete Antriebsordnung zur Erzeugung einer schrittweisen Bewegung der Behandlungs-Einzeleinheiten mit einer charakteristischen Anzahl von Haltestationen, in denen spezifische Behandlungsschritte ablaufen, zugeordnet, wobei Verbindungssysteme für die Prozeßmedien vorgesehen sind, die auf der einen Seite mit einer ortsfesten Versorgungseinheit fest verbunden sind und die auf der anderen Seite mit dem Zu- und Abfluß der Behandlungs-Einzeleinheiten haltestationsspezifisch in Wirkeingriff bringbar sind, so daß die Behandlungs-Einzeleinheiten in den Haltestationen von der ortsfesten Versorgungseinheit positionsabhängig mit den für die jeweilige Behandlungsstufe notwendigen Prozeßmedien beaufschlagbar sind.

Bei dem bevorzugten erfindungsgemäßen System wird zunächst in der ersten Haltestation eine Behandlungs-Einzeleinheit gebildet, beispielsweise eine bestimmte Menge von zu behandelnden Produkten in einer ersten Einzelkammer eingeschlossen. Danach wandert diese Einzeleinheit, beispielsweise die Einzelkammer, zur nächsten (zweiten) Haltestation, wo auch der nächste Verfahrensschritt durchgeführt wird, beispielsweise "Vorwärmen". Zur gleichen Zeit wird der Inhalt der nachfolgenden Einzeleinheit in der ersten Haltestation ausgeladen und mit neuem Gut bestückt. Im nächsten Zeittakt wandert besagte erste Einzeleinheit zur nächsten, der dritten Haltestation, die zweite Einzeleinheit zur zweiten Haltestation usw.. Nach vollständigem Durchlauf kann das behandelte Gut entnommen werden.

Bei dem erfindungsgemäßen System sind die Produkte während ihrer Behandlung in vielen kleinen Behandlungs-Einzeleinheiten untergebracht, d.h. auf eine Einzeleinheit entfallen entsprechend weniger Produkte als vergleichsweise beim einräumigen Großraumautoklaven. Die Behandlung erfolgt in jeder Einzeleinheit nach wie vor nach dem oben genannten Batchverfahren, für die Gesamtheit der Einzeleinheiten ergibt sich jedoch das kontinuierlichere Schieberegisterverfahren und für die Aggregate der Medienversorgung sogar eine quasi vollkontinuierliche Betriebsweise.

Die Produkte treten einzeln und regelmäßig in die Vorrichtung ein und verlassen sie nach der Behandlung mit gleicher Frequenz. Da sich die Produkte während ihrer Behandlung vorzugsweise in abschließbaren Kammern befinden, können Behandlungsverfahren angewendet werden, die die Abschließbarkeit relativ zur Anlagenumgebung erfordern. Zu diesen Verfahren zählen beispielsweise Behandlungen bei Unter- oder Überdruck oder Behandlungen mit Medien, die nicht in die Umgebung austreten dürfen.

Dieses Verfahren bringt folgende Vorteile:

Die erforderlichen Produktpuffer sind um mindestens den Faktor kleiner, der der Anzahl der Behandlungs-Einzeleinheiten, insbesondere der Einzelkammern entspricht, im Vergleich zu einem gleich produktiven Großraumautoklaven.

Die Anzahl der Produkte, die in einer Einzelkammer als Charge zusammen behandelt werden, ist gegenüber dem Großraumautoklav reduziert. Dadurch ist die Gleichmäßigkeit der Medienströmungsführung in Bezug auf die Produkte, d.h. generell die Reproduzierbarkeit der physikalischen Bedingungen in der Autoklav-Einzelkammer prinzipiell höher als beim Großraumautoklav. Es ergeben sich dadurch geringere Überdimensionierungen des Behandlungsverfahrens und damit erhöhte Produktivität bei verbesserter Produktqualität.

Die Effektivität wird höher, da kein Arbeitstaktverlust beim Ein- und Ausräumen der Chargen entsteht. Gleichzeitig hat man keinen Platzverlust wie im nicht ausgefüllten Großraumautoklaven.

Während beim Großraumautoklaven die Behandlungsabschnitte einzeln und zeitlich getrennt stattfinden, erfolgen bei der Erfindung alle Behandlungsabschnitte fortwährend und gleichzeitig, jedoch zeitlich versetzt. Da die zugehörigen Aggregate in der Versorgungseinheit kontinuierlich betrieben werden, fallen sie kleiner und billiger aus als beim Großraumautoklaven, wo die Aggregate immer nur zeitweilig, dann aber mit höherer Leistung, betrieben werden müssen. Bei regelungstechnisch gleichem Aufwand arbeiten die kontinuierlich betriebenen Aggregate bei der Erfindung gleichmäßiger und reproduzierbarer als die diskontinuierlich betriebenen Aggregate des Großraumautoklaven.

Ein wichtiger weiterer Vorteil der Erfindung ist der Umstand, daß sich bei vergleichbarem technischen Aufwand verfahrensabhängig der Energieverbrauch gegenüber dem Großraumautoklaven maßgebend reduzieren läßt. Dies hängt u.a. an den kleineren und kontinuierlich im Wirkungsgradoptimum betriebenen Aggregaten.

Ein weiterer Vorteil ist die erhöhte Produktivität bei gleichmäßigem Betrieb.

Durch die Tatsache, daß die Produkte einzeln und gleichmäßig der Anlage zugeführt und wieder entnommen werden können, ist es möglich, die erfindungsgemäße Vorrichtung pufferlos in eine Fertigungslinie zu integrieren, zum Beispiel zwischen Montage und Verpackung.

Ein wesentlicher Vorteil der Erfindung ist die praktische Realisierbarkeit der teilweisen Energierüchgewinnung bei Behandlungsverfahren, welche Abkühlungs- und Aufheizprozesse enthalten. Die Kühlmedien heizen sich beim Abkühlen der Produkte, Kammern und Rohrleitungen auf und können fortlaufend an einer anderen Behandlungsstation mit zum Aufheizen der Produkte eingesetzt werden. Beim Großraumautoklaven liegen die Aufheiz- und Abkühlvorgänge jedoch zeitlich auseinander und große Medien- und Energiemengen müßten bis zu ihrer Wiederverwendung zwischengespeichert werden, was entsprechend höhere Investitionskosten bei der Anlage zur Folge hätte.

Bei Störfällen, die sich auf die einzelne Behandlungs-Einzeleinheit, beispielsweise eine Kammer, beziehen, beispielsweise das Undichtwerden einer Tur, ist es ohne großen Steuerungsaufwand möglich, die betreffende Kammer bis zur Behebung des Schadens aus dem Kreis der mit Produkten bestückten Kammern auszuschließen. Die Anlage muß bei solchen Störanfällen also nicht unbedingt stillgesetzt werden und nur die Produkte der defekten Einzelkammer werden ggfls. zu Ausschuß.

Bei Störfällen, die sich auf einzelne Aggregate beziehen, beispielsweise der Ausfall eines Gebläses, werden nur die Produkte ggfls. zu Ausschuß, die die Behandlung mit diesem defekten Gebläse noch vor sich haben; die übrigen Produkte können ordnungsgemäß zu Ende behandelt und der Anlage entnommen werden. Da ein Aggregat meistens während seiner Einschaltphase defekt wird, werden beim Großraumautoklaven dagegen in der Regel alle in der Kammer befindlichen Produkte zu Ausschuß.

Die Kontinuität des Verfahrens wird bis zum völligen Wegfall der Puffer dadurch weiter gesteigert, daß Handlingmaschinen die Produkte einzeln oder in kleinen Gruppen aus der gerade geöffneten Kammer entnehmen bzw. in sie hineinlegen und diese Produkte in noch weiter verkleinerten Zeitabständen und in vereinzelter Form auf Transporteinrichtungen in die Autoklavanlage bzw. aus ihr heraus gelangen. Da die zu behandelnden Produkte ohnehin stückige Form haben, also Stück für Stück aus dem vorgeschalteten Fertigungsabschnitt ankommen und analog Stück für Stück im nachgeschalteten Fertigungsabschnitt gebraucht werden, kann die erfindungsgemäße Autoklavanlage im Hinblick auf die stückigen Produkte zu Recht als kontinuierlich arbeitender Autoklav bezeichnet werden.

Vorteilhaft gegenüber den bekannten kontinuierlich arbeitenden Autoklaven ist auch die Vielseitigkeit der Verwendung. Da jedes Produkt (oder eine kleine Produktpalette) in einer austauschbaren Behandlungs-Einzeleinheit, beispielsweise einer Kammer, untergebracht ist, ist es möglich, gleichzeitig verschiedene Produkte in verschiedenen Kammern zu sterilisieren (unabhängig davon, ob sie flüssig, fest, granuliert, offen, verpackt etc. sind). Jeder Kammer können auch im gleichen Takt verschiedene Behandlungs-, insbesondere Sterilisationsmedien zugeführt werden, da jede Kammer eine eigene Zuführleitung aufweist. Beispielsweise kann eine Kammer mit heißem Wasserdampf durchspült werden, eine folgende aber z.B. mit wässriger Zitronensäure. Dennoch muß nicht die ganze Anlage auf das andere Sterilisationsmedium umgeschaltet werden. Auch andere Arbeitsgänge könnten eingeschaltet werden, z.B. Beizen, Beschichten, Reinigen etc.

Die Erfindung gibt einen großen Anwendungsbereich für die behandelbaren Produkte vor.
- Die in der Anlage behandelbaren Produkte müssen in einer Form vorliegen bzw. in eine solche gebracht werden können, welche es möglich macht, sie mittels geeigneter Handlingvorrichtungen in die Kammern einzubringen und nach ihrer Behandlung herauszunehmen. Diese Forderung ist für fast alle Produkte erfüllbar; Beispiele: Flüssige oder granulatförmige Produkte in geschlossenen oder offenen, starren oder nachgiebigen Behältnissen, mechanisch instabile oder schwierig handhabbare Produkte in Produktständern oder in halboffenen, porösen oder geschlossenen Verpackungen.
- In vielen Fällen ist das geeignete Verpackungsbehältnis für das Produkt nicht nur die Voraussetzung für dessen Handlebarkeit, sondern unverzichtbarer Bestandteil des Behandlungsverfahrens; Beispiel: Sterilisation von medizinischen Schlauchsystemen in sterilisierdampfdurchlässiger und zugleich bakterienundurchlässiger Membranverpackung.
- In anderen Fällen stellt das Produkt oder dessen Verpackung bereits selber einen abschließbaren Behälter, das heißt einen Autoklaven, dar, in welchem der Behandlungsprozeß durchgeführt werden kann; Beispiel: Ein Hämodialysefilter stellt einen Behälter dar, welcher mittels durchströmenden Dampfes sterilisiert werden kann. In diesen Fällen werden nach einer Weiterbildung der Erfindung produktaufnehmende Kammern durch Konnektionseinrichtungen zu den Produkten hin ersetzt. Bei Produkten, die während ihrer Behandlung innerlich mit Medien beauschlagt werden, können diese Konnektionseinrichtungen zum Beispiel Rohrleitungskupplungen sein. Bei den Konnektionseinrichtungen kann es sich zum Beispiel aber auch um äußerlich an die Produktoberfläche angekoppelte Wärmeübertrager handeln.

Auch hinsichtlich der anwendbaren Behandlungsverfahren ist der Anwendungsbereich beachtlich groß.
- Die Erfindung ermöglicht es, jede Behandlungs-Einzeleinheit, beispielsweise Einzelkammer, nach dem gleichen Programm und mit hoher Reproduzierbarkeit mit unterschiedlichen Medien zu beaufschlagen.
- Diese Medien können zur direkten oder indirekten Einflußnahme auf die Produkte eingesetzt werden; Beispiele: direkte Einwirkung bakterientötender Gase, direkte Einwirkung trocknender Heißluft, indirekte Einwirkung durch Sattdampf, der an der Oberfläche der Produktverpackung hindurch erwärmt, indirekte Einwirkung durch beheizte Rohrschlangen, die zum Beispiel unter Vakuumbedingungen Strahlungswärme auf das Produkt übertragen.
- Nachfolgend sind einige Beispiele für anwendbare Behandlungsverfahren angeführt:
   Trocknungsverfahren (Vakuum, Strahlungswärme, Heißluft...)
   Sterilisationsverfahren (feuchte/trockene Hitze, Sterilisiergase)
   Thermische Verfahren (kochen, backen,, aushärten)
   Weitere Verfahren wie beizen, beschichten, reinigen....

Ein besonders günstiger Aufbau der Vorrichtung ergibt sich, wenn die Behandlungs-Einzeleinheiten, insbesondere die Einzelkammern, gleichartig ausgebildet sind. Dies erleichtert auch die Wartung der Anlage und senkt die Herstellungskosten.

Grundsätzlich können die Behandlungs-Einzeleinheiten, beispielsweise die Einzelkammern, in einer linearen Kette bewegt werden, mit einem Schnellrücklauf der Kammer, die den letzten Behandlungsschritt durchlaufen hat, zum Ausgangspunkt. Dabei ist es zweckmäßig, eine zusätzliche Einzelkammer vorzusehen und diese jeweils am Startpunkt in eine Parkposition zu bringen, um sie sofort in die Kette einkoppeln zu können, bevor die rücklaufende Kammer am Startpunkt ist, die dann ihrerseits die Parkposition einnimmt.

Besonders vorteilhaft ist es jedoch nach einer Ausgestaltung der Erfindung, wenn die Behandlungs-Einzeleinheiten, beispielsweise die Einzelkammern, kreisringförmig angeordnet sind und die getaktete Antriebsanordnung einen Drehantrieb aufweist, d.h. verfahrensmäßig das zu behandelnde Gut mit seiner Einzeleinheit umlaufend transportiert wird.

Dadurch wird einmal der Raumbedarf verhältnismäßig klein. Ferner wird durch die dann stattfindende Umlaufbewegung ein gleichförmiger, zyklischer Bewegungsablauf erzielt, der auch eine bessere Versorgbarkeit der bewegten Einzelkammern mit den behandelnden strömenden Medien aus dem Zentrum der Umlaufbewegung heraus mittels einer neuartigen Durcheinspeisung erlaubt.

Es ist aber auch der Aufbau einer Kette von Behandlungs-Einzeleinheiten denkbar, die stationär angeordnet sind, deren zeitlicher Behandlungsprozeß ortsspezifisch durch eine gemeinsame Steuereinheit bestimmt wird, wobei Bedienungspersonal an der Kette vorbeigeht, um die jeweilige Einzeleinheit zu be- und entladen.

Weitere ausgestaltete Merkmale, Vorteile sowie Anwendungsmöglichkeiten der Erfindung ergeben sich anhand von in den Zeichnungen dargestellten Ausführungsbeispielen der Erfindung.

Es zeigen:
- Figur 1: in schematischer, perspektivischer Darstellung eine Gesamtansicht einer vorteilhaften, bevorzugten Ausführungsform der erfindungsgemäßen Vorrichtung mit Einzelkammern als Behandlungs-Einzeleinheiten, die in Kreisringform angeordnet sind.
- Figur 2: einen Längsschnitt durch den Kreisring der Einzelkammern der Vorrichtung nach Figur 1.
- Figur 3: einen Längsschnitt durch eine Einzelkammer der Vorrichtung nach Figur 1.
- Figur 4: eine Frontansicht einer Einzelkammer bei geöffneter Tur.
- Figur 5: einen vergrößerten Ausschnitt aus Figur 2 im Bereich der Dreheinspeisung.
- Figur 6: einen vergrößerten Ausschnitt aus Figur 5 im Gleitbereich der Dreheinspeisung.
- Figur 7: eine Draufsicht auf den Stator der Dreheinspeisung.
- Figur 8: in schematischer Darstellung den räumlichen Programmschaltplan in der Dreheinspeisung.

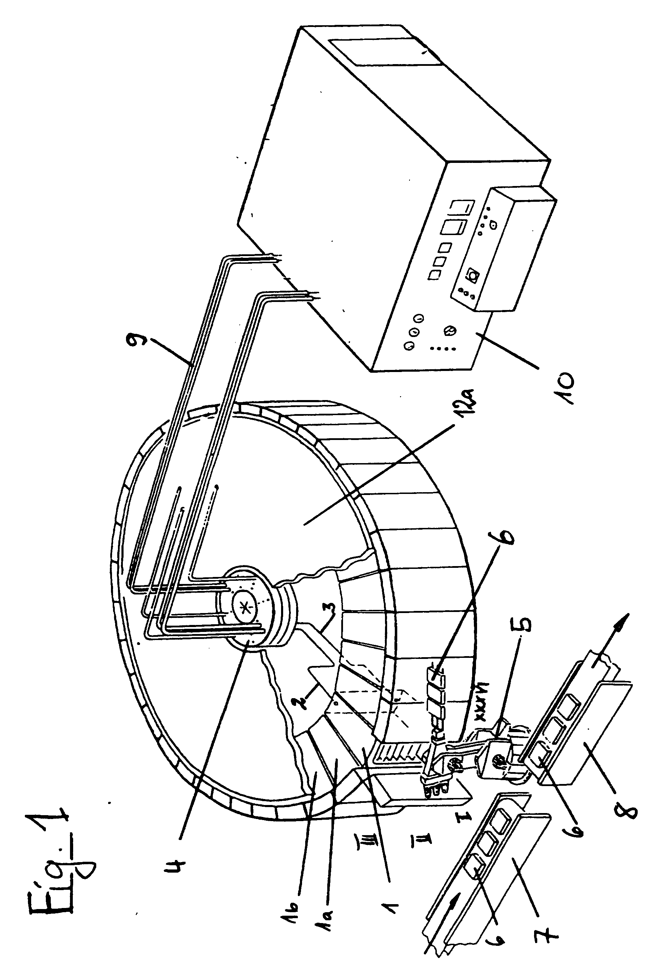

Figur 1 zeigt eine Autoklav-Vorrichtung zur mehrstufigen Behandlung von stückigen Produkten mittels strömender Medien.

Der Autoklav besteht aus einer Mehrzahl von Einzelkammern 1, 1a, 1b usw, wobei im vorliegenden Ausführungsbeispiel 36 Kammern vorgesehen sind. Die Zahl der Kammern bestimmt sich u.a. nach der Zahl der Behandlungsschritte, der notwendigen Pufferung, der Zahl der Zyklen, dem Raumangebot etc.

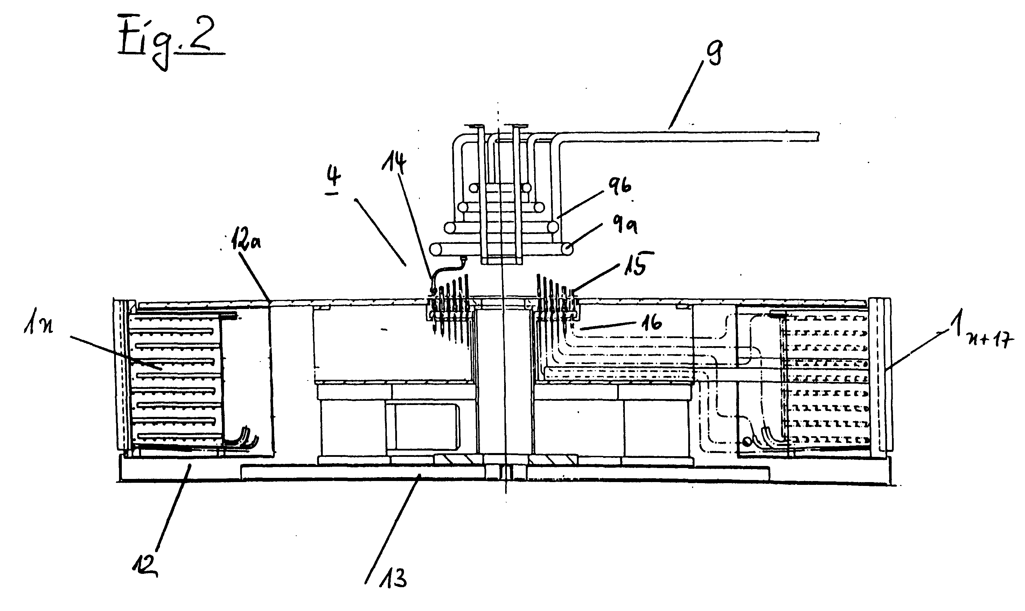

Die Einzelkammern sind kreisringförmig auf einem Drehgestell angeordnet und werden schrittweise, d.h. jeweils um eine Kammerbreite (1/36-Umdrehung), bewegt, wie anhand der Figur 2 noch näher erläutert wird.

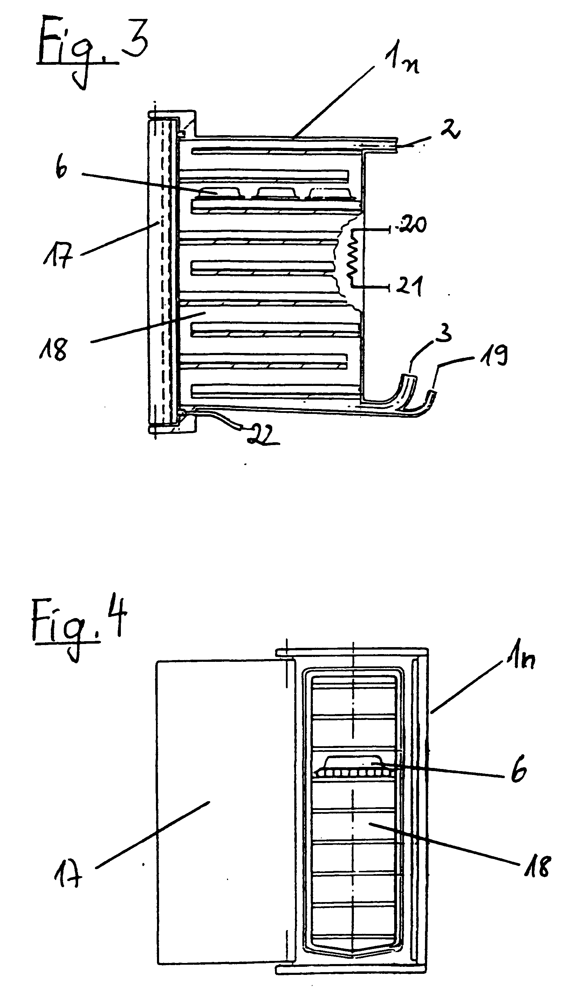

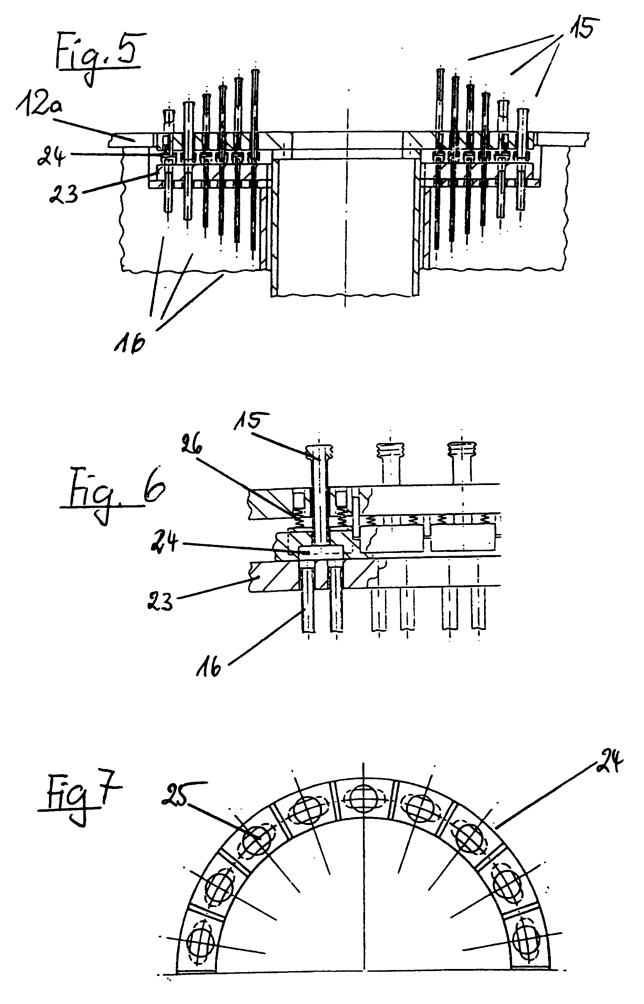

Jede Einzelkammer stellt einen im wesentlichen rechteckigen Druckbehälter mit dicht verschließbarer Tür 17 dar. Der prinzipielle Aufbau der Einzelkammern soll später anhand der Figuren 3 und 4 noch näher vertieft werden. Jede Einzelkammer weist einen Zu- und Abfluß 2, 3, für die Prozeßmedien und die Druck- bzw. Unterdruckerzeugung auf, die an eine zentrale Dreheinspeisung 4 geführt sind, deren Aufbau anhand der Figur 5 noch näher erläutert wird.

Jedem Kammerplatz, d.h. jeder Kammerposition ist eine Haltestation zugeordnet, in der positionsspezifische Prozeßabläufe stattfinden. In Figur 1 ist dabei der Haltestation I, in der sich gerade die Einzelkammer 1 befindet, das Beund Entladen der Kammern zugeordnet. Ein ortsfest installierter (nicht dargestellter) Mechnismus übernimmt hierbei das selbsttätige Öffnen und Schließen der Kammertür. Der Haltestation I, der Beladestation, ist ferner ein handelsübliches Handlinggerät 5 in Form eines Industrieroboters zum chargenweisen Ein- und Ausladen der Produkte 6 in die jeweilige in der Haltestation I befindliche Einzelkammer, in Figur 1 in die Kammer 1, zugeordnet.

An der Ladestation beginnt bzw. endet der Behandlungszyklus mit dem Ein- und Ausladen der Produkte, wobei eine Anlage mehrere solcher Haltestationen aufweisen kann. Eine Transporteinrichtung 7 bringt die zu behandelnden Produkte 6 von dem Außenbereich der Anlage zu der Ladestation, während eine Transporteinrichtung 8 die behandelten Produkte aus dem Bereich der Anlage herausbringt.

Das Handlinggerät 5 entnimmt beim Beladen der Einzelkammer die Produkte 6 einzeln oder in kleinen Gruppen (im Beispiel 3) von der Transporteinrichtung 7 bzw. übergibt in gleicher Weise beim Ausladen die Produkte 6 der Transporteinrichtung 8.

Das Be- und Entladen kann natürlich auch manuell erfolgen.

Wenn dabei das Be- und Entladen während der Dauer des zeitlich längsten Behandlungsschrittes, d.h. des Taktes, erfolgt, ergibt sich insgesamt eine kontinuierliche Arbeitsweise der Autoklav-Anlage.

In jeder der übrigen Haltestationen II, III etc bis XXXVI bleiben die Kammertüren geschlossen und die Einzelkammern werden positions-, d.h. haltestationsspezifisch über die Dreheinspeisung 4 und Verbindungssysteme 9 von einer ortsfesten Versorgungseinheit 10 mit unterschiedlichen Prozeßmedien beaufschlagt.

Die Versorgungseinheit faßt in einer gemeinsamen Einhausung die Medienperipherie und die Steuerung zusammen.

Die Medienperipherie umfaßt alle erforderlichen Aggregate zur Aufbereitung und Verteilung der Prozeß- und Hilfsmedien zwischen den Medienleitungen des Hausnetzanschlusses und den Medienleitungen zur Dreheinspeisung.

Unter den Begriff der Steuerung fallen alle Geräte zur Ablaufsteuerung, zur Schaltung von elektrischen und steuerpneumatischen Verbrauchern, Sensoren, Auswert- und Regelgeräte Betriebssoftware, Anlagenverkabelung und ein Bedienfeld mit seinen Schaltern, Anzeigegeräten und Schreibern.

Die Versorgungseinheit 10 und die Verbindungssysteme 9 sind aus bekannten Mitteln aufgebaut und können von einem Fachmann der Verfahrenstechnik ohne weitere Angaben in üblicher Weise aufgebaut und verschaltet werden.

Die Aggregate der Versorgungseinheit arbeiten kontinuierlich. Die zeitgerechte Beaufschlagung der Kammern mit dem richtigen Medium erfolgt über die Dreheinspeisung 4. Sie vermittelt den Übergang der Medien von der ortsfesten Versorgungseinheit 10 bzw. von dem daran fest angeschlossenen Verbindungssystem 9 auf die sich drehenden Einzelkammern. Die Dreheinspeisung besteht, wie insbesondere die Figur 5 zeigt, grundsätzlich aus zwei Teilen, nämlich einem Rotor in Form einer Gleitscheibe 23, der über die Leitungen 2, 3 starr mit den Einzelkammern 1, 1a, etc. verbunden ist und sich mit ihnen zusammen dreht und einem Stator in Form von Gleitringen 24, die fest mit den Verbindungssystemen 9 verbunden sind, d.h. ortsfest sind.

Die Gleitscheibe (der Rotor) weist für die Anschlüsse 2 und 3 jeder Einzelkammer ein Loch auf, über das die Medien in die Kammer ein- und ausströmen können. Die Gleitringe (der Stator) weisen für jeden Prozeßmedienanschluß der Verbindungssysteme 9 ein oder mehrere Langlochzonen 25 auf (Figur 7), um einen freien Durchgang zu den Löchern in der sich drehenden Gleitscheibe für den Durchtritt des Prozeßmediums zu schaffen. Die Lage der Löcher und der Langlochzonen ist dabei so gewählt, daß jede Einzelkammer in der jeweiligen Haltestation mit den dort benötigten Prozeßmedien versorgt bzw. in der notwendigen Weise aktiviert wird.

Die Dreheinspeisung enthält also baulich das Medienschaltprogramm, wodurch eine Steuerung mit Ventilen weitgehend entfällt.

Wegen der durch die Dreheinspeisung übernommenen Funktionen der zeitlichen und schaltungsmäßigen Mediensteuerung fällt der Aufwand für die Steuerung im Verhältnis zur Anlagengröße kleiner aus als beim Durchschnitt der Anlagen.

Die Verschaltung der Medienleitungen zwischen den Anschlußstutzen der Dreheinspeisung und den Rohrleitungen zur Versorgungseinheit erfolgt zweckmäßig weichenartig. Sollten in bestimmten Drehwinkelbereichen mehrere Einzelkammern in Reihe geschaltet werden, so legt man Rohrbögen von den Stutzen der Kammerausgänge zu den Stutzen der benachbarten Kammereingänge; will man dagegen mehrere Kammern parallel durchströmen, so führt man jeweils mehrere Anschlußstutzen zusammen und schaltet sie auf eine Hauptmedienleitung.

Die Figur 2 zeigt einen Schnitt durch die ringförmige Kammeranordnung nach Figur 1 an einer willkürlichen Stelle, d.h. durch die Einzelkammer 1n und die gegenüberliegende Einzelkammer 1ₙ₊₁₇. Die Einzelkammern ruhen auf einem Drehgestell 12, das von dem Rundtaktantrieb in Form eines Rundschalttisches 13 getragen und bewegt wird. Derartige Rundschalttische sind handelsüblich und gewährleisten die notwendigen Taktzeiten und die erforderliche Teilungsgenauigkeit bei dem schrittweisen Bewegen der Einzelkammern.

Der gesamte Innenraum des Kreisringes ist mit einer Abdeckung 12a (s. auch Figur 1) versehen, um einmal der Anlage ein geschlossenes äußeres Bild zu geben und um zum anderen die Anlagenteile zu schützen.

Die Leitungen der Verbindungssysteme 9 münden in Ringleitungen 9a, 9b etc., die ihrerseits über Schlauchleitungen 14 und Anschlußstutzen 15 mit den Gleitringen der Dreheinspeisung 4 verbunden sind. Die Anschlüsse der Kammern sind ihrerseits an Stutzen 16 geführt, die an dem Rotor der Dreheinspeisung bzw. an den darin befindlichen Löchern angebracht sind. Diese Leitungsführung ist in dem rechten Teil der Figur 2 schematisch dargestellt.

Die dargestellten Verbindungsarten sind nur beispielhaft. Hier stehen dem Fachmann die verschiedensten Möglichkeiten und Untervarianten zur Verfügung.

Die Figuren 3 und 4 zeigen als Ausführungsbeispiel Einzelheiten im Aufbau der Einzelkammern, die vorzugsweise gleichartig ausgebildet sind. Die Figur 3 zeigt dabei einen Längsschnitt durch die Einzelkammer 1ₙ bei geschlossener Tür 17, während die Figur 4 eine Frontansicht bei geöffneter Tür zeigt. Jede Einzelkammer stellt einen im wesentlichen rechteckigen Druckbehälter mit dicht verschließbarer Tür dar. Der Innenraum der Einzelkammer ist in Fächern 18 unterteilt, wobei auf dem Gefachboden jeweils ein Rippenblech aufliegt. Die Produkte 6, von denen eine Dreiergruppe gezeigt ist, liegen auf dem Rippenblech. Die Rippenbleche sind von Fach zu Fach abwechselnd versetzt angeordnet (s. Figur 3) und bewirken hierdurch eine mäanderförmige Strömungsführung der Prozeßmedien und die allseitige Umströmung der Produkte durch die Prozeßmedien. Außerdem ermöglichen es die Rippenbleche, die Produkte mittels gabelförmiger Greifer in die Fächer ein- und auszuladen.

Für die Konstruktion von Tür, Türrahmen, Türverschluß und Türdichtung stehen dem Fachmann eine Reihe von Möglichkeiten zur Verfügung, z.B. Klapptürkonstruktionen oder Schiebetüren mit Öffnungsrichtung nach oben.

Die Bauart des Türbetätigungsmechanismus ergibt sich für den Fachmann durch die gewählte Bauart der Türen.

Jede Einzelkammer besitzt Anschlüsse 2, 3, 19-22, die, wie die Figur 2 (in Verbindung mit der Figur 5) schematisch zeigt, an die Gleitscheibe 23 der Dreheinspeisung 4 geführt sind. Die beiden großlumigen Anschlüsse 2 und 3 dienen als Prozeßanschlüsse für die Prozeßmedien. Der kleinlumige Prozeßanschluß 19 hat die Aufgabe der Ableitung von Kammerkondensat. Die Anschlüsse 20 und 21 versorgen die Rohrschlangenheizungen der einzelnen Fächer mit Heizdampf und leiten das anfallende Kondensat ab. Der Anschluß 22 versorgt die Türbetätigung, beispielsweise durch Druckluft.

Die Figuren 5-7 zeigen Einzelheiten der Dreheinspeisung 4 der Figuren 1 und 2. Dabei stellt die Figur 5 einen vergrößerten Schnitt durch das (obere) Zentrum der Figur 2 dar. Die Figur 6 ist ein vergrößerter Ausschnitt aus der Figur 5 im Bereich des Zusammenwirkens zwischen Rotor und Stator der Dreheinspeisung.

Die Figur 7 wiederum zeigt eine Draufsicht auf einen Gleitring des Stators.

Die Stutzen 16, an die die Anschlüsse der Einzelkammern (Figur 3) geführt sind (s. auch Figur 2, rechter Teil) münden in die Gleitscheibe 23 bzw. in den darin befindlichen Bohrungen. Die Stutzen 15, die gemäß Figur 2 über Schlauchleitungen 14 mit den Ringleitungen 9a ff. der Verbindungssysteme 9 verbunden sind, münden in Gleitringe 24 bzw. in darin befindliche Langlochzonen 25, die in der Draufsicht eines Gleitringes besonders gut aus Figur 7 zu erkennen sind. Federvorgespannte Bauelemente 26 sorgen für den notwendigen Andruck des Rotors am Stator, damit die Prozeßmedien nicht seitlich austreten können. Es ist dabei zweckmäßig, in diesem Gleitbereich Dichtungssysteme vorzusehen, wofür dem Fachmann verschiedene Möglichkeiten zur Verfügung stehen.

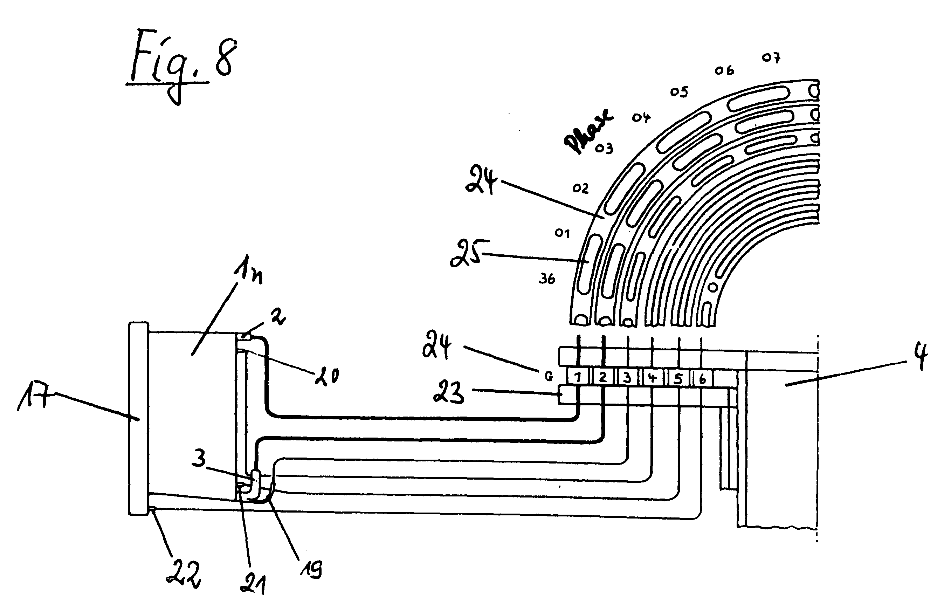

Die Figur 8 zeigt zur näheren Erläuterung der "Verschaltung" der Kammern mit den Verbindungssystemen 9 über die Dreheinspeisung einen "Schaltplan" für das in der nachfolgenden Tabelle beschriebene Verfahren. Die Figur 8 zeigt dabei eine Einzelkammer 1ₙ mit Tür 17 und Anschlüssen 2, 3 und 19-22 (s. Figur 3), die mit der Gleitplatte 23 fest verbunden sind. Es sind sechs Gleitringe 24 dargestellt und mit G1-G6 bezeichnet. Im oberen Teil der Figur 8 sind diese Gleitringe mit ihren Langlochzonen 25 als Draufsicht quasi hochgeklappt, mit der Zuordnung der einzelnen Langlochzonen zu den Behandlungsschritten, Phasen genannt. Diese Phasen entsprechen letztlich auch den einzelnen Haltestationen.

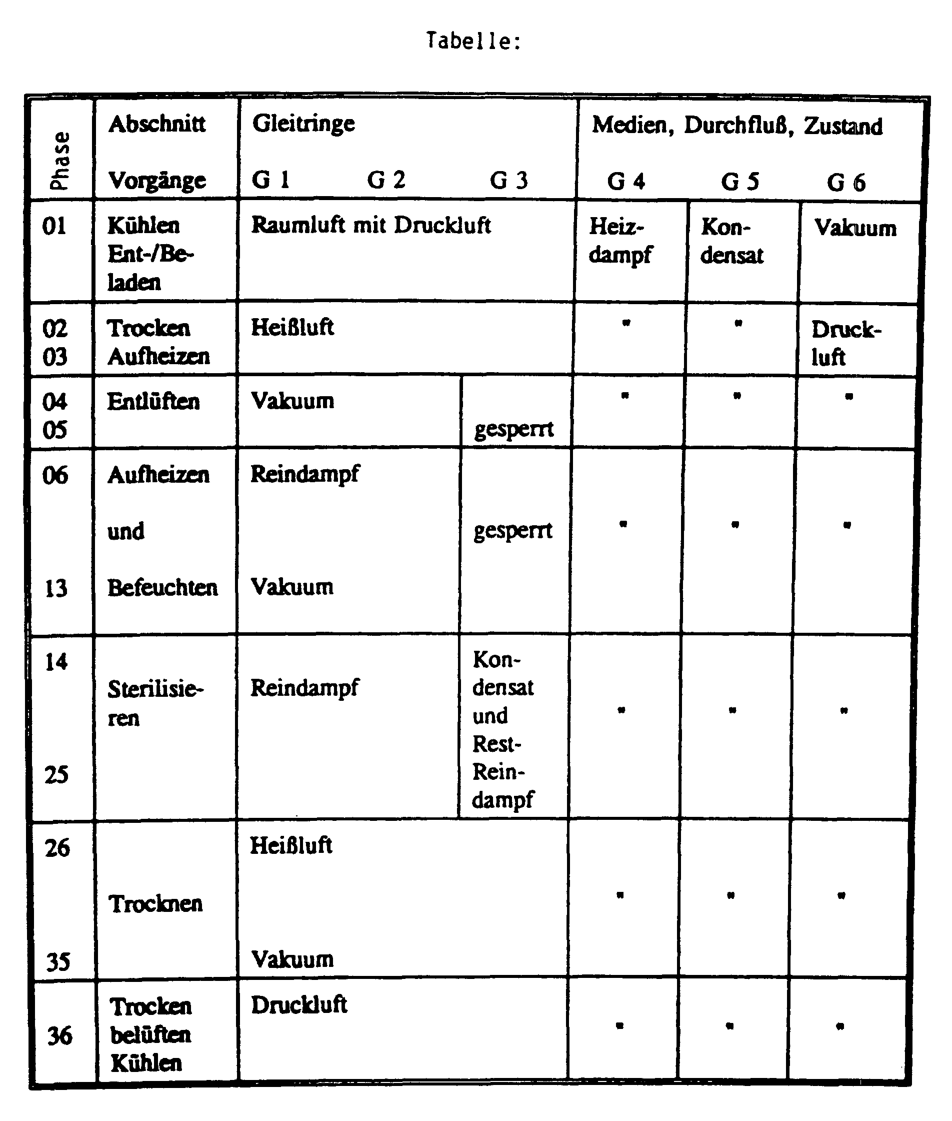

Die Tabelle zeigt in der ersten Spalte die Phasen, insgesamt entsprechend der im Ausführungsbeispiel gewählten Kammerzahl 36 Phasen. Die zweite Spalte zeigt, welche Behandlungs- bzw. Verfahrensschritte den einzelnen Phasen zugeordnet sind. Aus den weiteren Spalten ist zu entnehmen, welche Prozeßmedien bei den einzelnen Verfahrensschritten über die Langlochzonen der Gleitringe - und damit entsprechend Figur 8 - in die zugehörigen Anschlüsse der Einzelkammern - strömen.

So wird in der Phase 01, d.h. in der Beladestation I (s. Figur 1) über den Gleitring 6 am Anschluß 22 (s. Figur 8) ein Vakuum erzeugt, d.h. die Kammertür 17 öffnet sich. Gleichzeitig strömt über die Gleitringe G1-G3, d.h. in die Kammeranschlüsse 2, 3 und 19, Raumluft mit Druckluft ein bzw. aus. Über den Gleitring G4 wird Heizdampf auf den Anschluß 20 der Rohrschlangenheizung geschaltet; das Kondensat wird über den Anschluß 21 und den Gleitring G5 abgezogen.

In der zweiten Phase, wenn sich also die Einzelkammern um einen Schritt bewegt haben, findet (in der betrachteten Kammer) das Trocknen und Aufheizen der Produkte und der Kammer statt, und zwar mit Heißluft, die über die Gleitringe G1-G3 eingespeist und abgezogen wird. Die Rohrschlangenheizung wird weiterhin über die Gleitringe G4 und G5 beschickt. Die Kammertür ist in dieser Phase geschlossen, da über den Gleitring G6 Druckluft am Türstutzen 22 ansteht. Diese Vorgänge wiederholen sich, wenn die Kammern wiederum einen Schritt in die Phase 3 weitergedreht werden, d.h. der entsprechende Verfahrensschritt wird auf zwei Kammerschritte verteilt. Entsprechend laufen die anderen Phasen ab, bis schließlich mit der Phase 36, dem "Trocken belüften und Kühlen", das Ende des Zyklus erreicht ist und die Produkte zum Entladen bereit stehen.

In einer besonders vorteilhaften Ausführungsform weist die Vorrichtung nach Figur 1 nicht nur austauschbare Kammern auf, sondern Konnektoranschlüsse, so daß während eines Sterilisationsvorganges z. B. Dialysatoren angeschlossen werden können sowie Kammern mit Schlauchsystemverpackungen. Durch die Vielseitigkeit der Anschlußmöglichkeiten ist die Verwendungsmöglichkeit universell.

## Patentansprüche

1. Verfahren zum Behandeln von stückigen Produkten mittels Prozeßmedien im Rahmen einer Abfolge von Verfahrensschritten (Behandlungsstufen) in einer Behandlungseinheit, mit den Schritten:
- als Behandlungseinheit wird eine kreisringförmige Kette von Einzelkammern gebildet, in denen die stückigen Produkte in einer kleinen Charge aufgenommen sind und für die Dauer der Abfolge der Verfahrensschritte verbleiben,
- in der Kette, aufgeteilt auf die getrennten Einzelkammern, laufen, die einzelnen Verfahrensschritte der Abfolge gleichzeitig ab,
- die Abfolge der Verfahrensschritte in den Einzelkammern der Kette beziehungsweise in Gruppen davon, beginnt in einem vorgegebenen Takt zeitlich versetzt, und
- der zeitliche Versatz (Takt) wird von einer gemeinsamen Steuer- beziehungsweise Regeleinheit vorgegeben.

2. Verfahren nach Anspruch 1, **dadurch gekennzeichnet, daß** die gemeinsame Steuereinheit mechanisch in Form eines Transportsystems für die Einzelkammern gebildet wird, das die Einzelkammern an verschiedene Orte transportiert, in denen jeweils mindestens eine Behandlungsstufe durchgeführt wird, und daß der Beginn der Behandlung durch den Ort der jeweiligen Einzelkammer festgelegt wird.

3. Verfahren nach Anspruch 1, **dadurch gekennzeichnet, daß** die Einzelkammern während des Behandelns weitertransportiert werden und der zeitliche Versatz des Beginns der Behandlung zusätzlich durch eine gemeinsame Steuerbeziehungsweise Regeleinheit für die Prozeßmedien bestimmt wird.

4. Verfahren nach Anspruch 2, **dadurch gekennzeichnet, daß** die Einzelkammern nacheinander schrittweise an die Behandlungsorte transportiert werden, und daß die Kette von Einzelkammern gebildet wird, indem jeweils eine neue Einzelkkammer geladen wird, wenn die vorangehende, geladene Einzelkammer in die nächste Behandlungsstufe transportiert sowie eine behandelte Einzelkammer ausgeladen wird, wenn die nachfolgende Einzelkammer zur letzten Behandlungsstufe transportiert worden ist.

5. Vorrichtung zum Behandeln von stückigen Produkten (6) mittels einer Versorgungseinheit (10) entnehmbaren Prozeßmedien im Rahmen einer Abfolge von Verfahrensschritten (Behandlungsstufen) in einer Behandlungseinheit, **dadurch gekennzeichnet,**
- **daß** die Behandlungseinheit aus einer Mehrzahl von mindestens drei separaten, kreisringförmig in einer Kette angeordneten Einzelkammern (1, 1a, 1b) besteht, die jeweils mindestens einen Zu- und Abfluß (2, 3) für die Prozeßmedien aufweisen, in denen die stückigen Produkte (6) in einer kleinen Charge aufgenommen sind und für die Dauer der Abfolge der Verfahrensschritte verbleiben,
- **daß** die Zu- und Abflüsse (2, 3) der Einzelkammern mit der Versorgungseinheit (10) so verbunden sind, daß in der Kette, aufgeteilt auf die einzelnen Einzelkammem, die einzelnen Verfahrensschritte der Abfolge gleichzeitig ablaufen,
- **daß** eine gemeinsame Steuer- beziehungsweise Regeleinheit (12, 13) vorgesehen ist, die derart aufgebaut und mit den Einzelkammern (1, 1a, 1b) verbunden ist, daß die Abfolge der Verfahrensschritte in den Einzelkammern der Kette beziehungsweise in Gruppen davon, in einem vorgegebenen Takt zeitlich versetzt beginnt.

6. Vorrichtung nach Anspruch 5, **dadurch gekennzeichnet, daß**
- den Einzelkammern (1, 1a, 1b) als gemeinsame Steuereinheit eine getaktete Antriebsordnung (12, 13) zur Erzeugung einer schrittweisen Bewegung der Einzelkammern mit einer charakteristischen Anzahl von Haltestationen (I, II, III), in denen spezifische Behandlungsschritte ablaufen, zugeordnet ist, und
- Verbindungssysteme (9) für die Prozeßmedien vorgesehen sind, die auf der einen Seite mit einer ortsfesten Versorgungseinheit (10) fest verbunden sind und die auf der anderen Seite mit dem Zu- und Abfluß (2, 3) der Einzelkammern (1, 1a, 1b) haltestationsspezifisch in Wirkeingriff bringbar sind, so daß die Einzelkammern in den Haltestationen von der ortsfesten Versorgungseinheit positionsabhängig mit den für die jeweilige Behandlungsstufe notwendigen Prozeßmedien beaufschlagbar sind.

7. Vorrichtung nach Anspruch 5 oder 6, **dadurch gekennzeichnet, daß** die Einzelkammern (1, 1a, 1b) kreisringförmig auf einem Drehgestell (12) montiert angeordnet sind und die getaktete Antriebsordnung ein Drehantriebssystem (12, 13) aufweist.

8. Vorrichtung nach Anspruch 7, **dadurch gekennzeichnet, daß** zur positionsabhängigen Beaufschlagung der Einzelkammern (1, 1a, 1b) mit Prozeßmedien eine Dreheinspeisung (4) vorgesehen ist, die einmal einen Stator (24) mit darin befindlichen Öffnungen (25) aufweist, der starr mit den Verbindungssystemen (9) verbunden ist, und der zum anderen einen Rotor (23) mit darin befindlichen Öffnungen aufweist, der starr mit dem Drehgestell (12) verbunden ist und an den die Zu- und Abflüsse (2, 3) der Einzelkammern geführt sind, und daß die Öffnungen im Stator und Rotor in ihrer räumlichen Lage so aufeinander abgestimmt sind, daß drehwinkelabhängig jeweils die für die Behandlung notwendige Prozeßleitung der Verbindungssysteme (9) mit den Zu- und Abflüssen der in den betreffenden Haltestationen befindlichen Einzelkammern verbunden ist.

## Claims

1. Process for the treatment of lump products by means of process media in the framework of a sequence of process steps (treatment steps) in a treatment unit, with the steps:
- As a treatment unit, a circular chain of individual chambers is formed, in which the lump products are taken up in a small batch and remain so for the duration of the sequence of process steps;
- In the chain, divided over the separate individual chambers, the individual process steps of the sequence take place simultaneously;
- The sequence of the process steps in the individual chambers of the chain, or in groups thereof begins in a specified cycle with temporal offsetting, and;
- The temporal offsetting (cycle) is specified by a common control or regulating unit.

2. Process according to Claim 1, **characterised in that** the common control unit is established mechanically in the form of a transport system for the individual chambers, that the individual chambers are transported to different locations, in which at least one treatment stage is carried out, and that the beginning of the treatment is specified by the location of each individual chamber.

3. Process according to Claim 1, **characterised in that** the individual chambers are transported further during treatment, and the temporal displacement of the beginning of the treatment is additionally determined by the common control or regulating unit for the process media.

4. Process according to Claim 1, **characterised in that** the individual chambers are transported stepwise one after another to the treatment locations, and that the chain of individual chambers is formed such that, in each case, a new individual chamber is loaded when the previous loaded individual chamber is transported into the next treatment step, and a treated individual chamber is unloaded when the following individual chamber has been transported to the last treatment step.

5. Device for the treatment of lump products (6) by means of process media derived from a supply unit (10) in the framework of a sequence of process steps (treatment steps) in a treatment unit, **characterised in that**:
- The treatment unit consists of a plurality of at least three separate individual chambers (1, 1a, 1b) arranged in circular fashion in a chain, which in each case features at least one inflow and outflow (2, 3) for the process media, in which the lump products (6) are taken up in the form of a small batch and remain so for the duration of the sequence of process steps.
- The inflows and outflows (2, 3) of the individual chambers are connected to the supply unit (10) in such a way that in the chain, divided over the individual chambers, the individual process steps of the sequence take place simultaneously;
- A common control or regulating unit (12, 13) is provided for, which is designed and connected to the individual chambers (1, 1a, 1b) in such a way that the sequence of process steps in the individual chambers of the chain or groups thereof begins temporally offset in a predetermined cycle.

6. Device according to Claim 5, **characterised in that**:
- As the common control unit, a cyclic drive arrangement (12, 13) is allocated to the individual chambers (1, 1a, 1b) for the generation of a stepped movement of the individual chambers with a characteristic number of stopping stations (I, II, III), in which specific treatment steps take place, and;
- Connection systems (9) for the process media are provided for, which on one side are permanently connected to a fixed-location supply unit (10), and which, on the other side, are capable of being brought into operational engagement with the inflow and outflow (2, 3) of the individual chambers (1, 1a, 1b) in a manner specific to the stopping stations, so that the individual chambers are capable of being imposed, in the stopping stations, from the fixed-position supply unit, in a position-dependent manner with the process medium necessary for the individual treatment step in question.

7. Device according to Claim 5 or 6, **characterised in that** the individual chambers (1, 1a, 1b) are arranged mounted in circular fashion on a rotating dolly (12), and the cyclic drive arrangement features a rotary drive system (12, 13).

8. Device according to Claim 7, **characterised in that**, for the position-dependent imposition of the individual chambers (1, 1a, 1b) with process media, a rotary infeed device (4) is provided for, which features on the one hand a stator (24) with apertures (25) located therein, which is rigidly connected to the connection systems (9), and which on the other hand also features a rotor (23) with the apertures located therein, which is rigidly connected to the rotating dolly (12) and is guided at the inflows and outflows (2, 3) of the individual chambers, and that the apertures in the stator and rotor are matched to one another in their spatial location in such a way that, dependent on the angle of rotation, in each case the process line of the connection systems (9), required for the treatment, is connected to the inflows and outflows of the individual chambers located in the stopping stations concerned.

## Revendications

1. Procédé pour le traitement de produits en morceaux à l'aide de milieux de processus industriels dans le cadre d'une succession d'étapes opératoires (étapes de traitement) dans une unité de traitement, comprenant les étapes consistant à :
- à titre d'unité de traitement, on forme une chaîne de chambres individuelles sous la forme d'un anneau de cercle, chambres dans lesquelles viennent se loger les produits en morceaux en formant une petite charge et y restent pendant la durée de la succession des étapes opératoires,
- dans la chaîne, subdivisée en chambres individuelles séparées, les étapes opératoires individuelles de la succession se déroulent de manière simultanée,
- la succession des étapes opératoires dans les chambres individuelles de la chaîne respectivement dans des groupes de ces dernières, commence de manière décalée dans le temps, dans un cycle prédéfini, et
- le décalage dans le temps (le cycle) est prédéfini par une unité commune de commande respectivement de réglage.

2. Procédé selon la revendication 1, **caractérisé en ce que** l'unité de commande commune est réalisée, du point de vue mécanique, sous la forme d'un système de transport pour les chambres individuelles, qui transporte les chambres individuelles à différents endroits dans lesquels on effectue respectivement au moins une étape de traitement, et **en ce que** le début du traitement est déterminé par l'endroit de la chambre individuelle respective.

3. Procédé selon la revendication 1, **caractérisé en ce que** les chambres individuelles sont transportées plus loin au cours du traitement et le décalage dans le temps du début du traitement est en outre déterminé par une unité commune de commande respectivement de réglage pour les milieux de processus industriels.

4. Procédé selon la revendication 2, **caractérisé en ce que** les chambres individuelles sont transportées successivement pas à pas aux endroits de traitement et **en ce que** la chaîne est formée par des chambres individuelles, en chargeant respectivement une nouvelle chambre individuelle lorsque la chambre individuelle chargée précédente a été transportée à l'étape de traitement suivante et en déchargeant une chambre individuelle traitée lorsque la chambre individuelle suivante a été transportée en direction de la dernière étape de traitement.

5. Dispositif pour le traitement de produits en morceaux (6) à l'aide de milieux de processus industriels qui peuvent être prélevés d'une unité d'alimentation (10) dans le cadre d'une succession d'étapes opératoires (étapes de traitement) dans une unité de traitement, **caractérisé**
- **en ce que** l'unité de traitement est constituée par une multitude d'au moins trois chambres individuelles (1, 1a, 1b) séparées, disposées en une chaîne sous la forme d'un anneau de cercle, chambres qui présentent respectivement au moins une entrée et une sortie (2, 3) pour les milieux de processus industriels, dans lesquelles les produits en morceaux (6) viennent se loger en formant une petite charge et y restent pendant la durée de la succession des étapes opératoires,
- **en ce que** les entrées et les sorties (2, 3) des chambres individuelles sont reliées à l'unité d'alimentation (10) de telle sorte que, dans la chaîne subdivisée en chambres individuelles séparées, les étapes opératoires individuelles de la succession se déroulent de manière simultanée,
- **en ce qu'**on prévoit une unité commune de commande respectivement de réglage (12, 13) qui est conçue et qui est reliée aux chambres individuelles (1, 1a, 1b) de telle sorte que la succession des étapes opératoires dans les chambres individuelles de la chaîne respectivement dans des groupes de ces dernières, commence de manière décalée dans le temps, dans un cycle prédéfini.

6. Dispositif selon la revendication 5, **caractérisé en ce que**
- on attribue aux chambres individuelles (1, 1a, 1b), à titre d'unité de commande commune, un ordre de commande cyclique (12, 13) pour l'obtention d'un mouvement pas à pas des chambres individuelles avec un nombre caractéristique de postes d'arrêt (I, II, III), dans lesquelles se déroulent des étapes de traitement spécifiques, et
- on prévoit des systèmes de liaison (9) pour les milieux de processus industriels qui d'une part,0 sont reliés à demeure à une unité d'alimentation fixe (10) et qui d'autre part, peuvent être amenés en engrènement de travail, de manière spécifique à un poste d'arrêt, avec l'entrée et la sortie (2, 3) des chambres individuelles, de telle sorte que les chambres individuelles dans les postes d'arrêt peuvent être chargées avec les milieux de processus industriels requis pour l'étape de traitement respective, en fonction de la position par rapport à l'unité d'alimentation fixe.

7. Dispositif selon la revendication 5 ou 6, **caractérisé en ce que** les chambres individuelles (1, 1a, 1b) sont disposées sous la forme d'un anneau de cercle en étant montées sur une plate-forme tournante (12) et l'ordre de commande cyclique présente un système de commande rotative (12, 13).

8. Dispositif selon la revendication 7, **caractérisé en ce que**, pour le chargement, qui dépend de la position, des chambres individuelles (1, 1a, 1b) avec des milieux de processus industriels, on prévoit une alimentation rotative (4) qui présente d'une part, un stator (24) dans lequel sont pratiquées des ouvertures (25) qui sont reliées à demeure aux systèmes de liaison (9) et qui présente d'autre part, un rotor (23) dans lequel sont pratiquées des ouvertures qui sont reliées à demeure avec la plate-forme tournante (12) et qui mènent aux entrées et sorties (2, 3) des chambres individuelles, et **en ce que** les ouvertures pratiquées dans le stator et dans le rotor sont adaptées l'une par rapport à l'autre en ce qui concerne leur position dans l'espace de telle sorte que, en fonction de l'angle de rotation, respectivement le contrôle des processus industriels des systèmes de liaison (9), requis pour le traitement, est relié aux entrées et aux sorties des chambres individuelles se trouvant dans les postes d'arrêt correspondants.
